(19) Europäisches Patentamt
European Patent Office
Office européen des brevets

(11) Numéro de publication: **0 349 402**
**A2**

(12) # DEMANDE DE BREVET EUROPEEN

(21) Numéro de dépôt: 89401801.9

(22) Date de dépôt: 26.06.89

(51) Int. Cl.5: C 07 K 1/04

(30) Priorité: 27.06.88 FR 8808611

(43) Date de publication de la demande:
03.01.90 Bulletin 90/01

(84) Etats contractants désignés:
AT BE CH DE ES FR GB GR IT LI LU NL SE

(71) Demandeur: RHONE-POULENC NUTRITION ANIMALE
Rue Marcel Lingot
F-03600 Commentry (FR)

(72) Inventeur: Blondelle, Sylvie
3056 A Via Alicante Drive
La Jolla CA 92037 (US)

Brugidou, Jean
8 rue Jacques Boe, La Paillade
F-34100 Montpellier (FR)

Commeyras, Auguste
6 Impasse des Ecoles
F-34170 Clapiers (FR)

Mion, Louis
477 rue d'Alco
F-34000 Montpellier (FR)

Taillades, Jacques
9 rue des Erables Lotissement Le Vert Pré
F-34170 Clapiers (FR)

(74) Mandataire: Pilard, Jacques et al
RHONE-POULENC SANTE Service Brevets Santé 25 Quai Paul Doumer
F-92408 Courbevoie Cédex (FR)

(54) **Procédé de préparation de polypeptides en phase solide.**

(57) La présente invention concerne un procédé de préparation de polypeptides en phase solide comportant les étapes successives consistant à :

(i) Faire réagir un polymère carbonylé avec un dipeptide pour fixer ce dernier sur ledit polymère sous la forme de cycles imidazolidinones ;

(ii) Activer la fonction acide terminale portée par lesdits cycles imidazolidinones fixés sur le polymère ; et

(iii) Coupler les fonctions acides terminales activées portées par les cycles imidazolidinones du polymère issu de l'étape précédente, par réaction de ce dernier avec un dérivé peptidique à fonction amine libre et à fonction carboxyle protégée, puis

(iv) Hydrolyser le polymère issu de l'étape précédente pour séparer le polypeptide et régénérer simultanément le polymère carbonylé de départ.

EP 0 349 402 A2

**Description**

## PROCEDE DE PREPARATION DE POLYPEPTIDES EN PHASE SOLIDE

La présente invention concerne un procédé de préparation de polypeptides en phase solide.

La synthèse peptidique en phase solide, introduite en 1963 par MERRIFIELD (J. Am. Chem. Soc., 85, 2149 (1963), a provoqué un véritable essor dans la chimie des peptides. L'avantage essentiel de la synthèse en phase solide réside dans la facilité avec laquelle les intermédiaires peuvent être obtenus exempts de tous réactifs en excès et/ou de sous-produits réactionnels. L'intermédiaire réactionnel se trouve en effet fixé sur le polymère insoluble, ce qui permet, par des lavages avec des solvants appropriés, d'éliminer les diverses impuretés. Les problèmes liés au choix des solvants solubilisant des produits de poids moléculaire élevé sont également évités. Il faut en effet utiliser des solvants permettant un bon gonflement de la résine. De plus, l'élimination des problèmes d'isolation des intermédiaires permet une automatisation du procédé.

A l'origine, cette technique antérieure a utilisé des polystyrènes préalablement activés par une chlorométhylation. Le carbone terminal de l'acide aminé N-protégé est fixé sur le support par l'intermédiaire d'un ester benzylique. Ces liaisons ester restent stables durant toute la synthèse peptidique. D'autres groupes fonctionnels ont été utilisés par la suite, tels que des groupes phénylacyle, hydrazyle, acylsulfonyle, benzhydryle, aminométhyle, etc. (Barany G., Merrifield R.B., Solid phase peptide synthesis in The Peptides (Eds Gross E., Meienhofer J.) Vol. 2, Academic Press, New-York, 1979, p.3). Cependant, il s'est avéré que les vitesses d'incorporation des aminoacides diminuaient au fur et à mesure de l'avancement de la synthèse. SHEPPARD montra que cette baisse de réactivité était due à une incompatibilité physicochimique entre la matrice polystyrénique et le peptide fixé (sheppard R.C. in : Peptides 1971 (Ed. Nesvadba H.) p.111, North Holland Publ., Amsterdam 1973). En effet, le support polymérique a un caractère hydrophobe non compatible avec les chaînes peptidiques hydrophiles. Pour pallier ce problème, des supports polymériques polaires ont été proposés tels que les polyacrylamides (Atherton E., Sheppard R.C., in Peptides 1974 (Ed. Wolman Y) Wiley, New-York 1975, p. 123) et polyacryloylpyrrolidines (Smith C.W., Stahl G.L., Walter R., Int. J. Pept. Protein Res 13, 109 (1979). Ces polymères possèdent les qualités mécaniques requises et une structure plus proche des peptides que les polystyrènes, la fonctionnalisation de ces résines se faisant lors de la polymérisation. Les taux de fixation des polyacryloylpyrrolidines (0,7 meq/g) sont légèrement supérieurs à ceux obtenus avec les polyacrylamides (0,35 meq/g) et largement compétitifs vis-à-vis des polystyrènes (0,1 à 0,3 meq/g). De plus, ces deux nouveaux types de résine ont des capacités de gonflement supérieures aux polystyrènes. En effet, dans le cas des résines polystyréniques, le gonflement est minimum dans des solvants très polaires tels que le méthanol et fortement augmenté dans des solvants chlorés (Wieland T., Birr C., Flor F., Liebigs Ann. Chem. 727, 130 (1969). Tandis que dans le cas des polyacryloylpyrrolidines, ce taux est important dans la plupart des solvants (Stahl G.L., Walter R., Smith C.W., J. Am. Chem. Soc. 101, 5383 (1979).

La présente invention a précisément eu pour but d'améliorer un tel procédé de préparation de polypeptides en phase solide.

Conformément à la présente invention, le procédé est caractérisé par le fait que l'on réalise successivement les étapes consistant a :

(i) Faire réagir un polymère carbonylé avec un dipeptide pour fixer ce dernier sur ledit polymère sous la forme de cycles imidazolidinones ;

(ii) Activer la fonction acide terminale portée par lesdits cycles imidazolidinones fixés sur le polymère ; et

(iii) Coupler les fonctions acides terminales activées portées par les cycles imidazolidinones du polymère issu de l'étape précédente, par réaction avec un dérivé peptidique à fonction amine libre et à fonction carboxyle protégée, puis

(iv) Hydrolyser le polymère issu de l'étape précédente pour séparer le polypeptide et régénérer simultanément le polymère carbonylé de départ.

Le polymère carbonylé, utilisé dans le cadre de la présente invention, peut avantageusement être préparé de la manière décrite dans FR-A-2 519 973.

Ces polymères carbonylés ont un caractère hydrophile compatible avec les chaînes peptidiques et présentent des propriétés mécaniques parfaitement satisfaisantes permettant leur mise en oeuvre aisée. Ils sont en outre très faciles d'accès. On notera enfin que leur taux de gonflement dans les différents solvants utilisés en synthèse peptidique ainsi que dans l'eau sont comparables a ceux des polyacryloylpyrrolidines. Enfin, leur forte capacité ($C_{pc=o} \simeq 4$ meq./g) leur permet des taux de fixation nettement plus élevés que ceux des polymères décrits dans l'art antérieur. L'ancrage des peptides se faisant par l'intermédiaire des imidazolidinones, ne nécessite pas d'activation préalable ou lors de la polymérisation des supports. L'aspect le plus positif de ces supports sont les conditions douces de fixation et de décrochage du peptide du support polymérique, ainsi que le fait qu'il soit automatiquement régénéré lors du décrochage.

De plus, de façon inattendue, il a été observé que le motif cétonique influe sur la basicité de la fonction amine de l'imidazolidinone fixée sur le support. A titre d'exemple, les imidazolidinones formées sur le polyacryloylpipéridone ont un pKa de l'ordre de 3, tandis que celles formées sur le polyacryloylcyclohexanone ont un pka de l'ordre de 5. Cet écart important de pKa permet de réaliser deux stratégies de synthèse peptidique, sans avoir à introduire de groupes protecteurs supplémentaires. Ainsi, en choisissant des polymères carbonylés conduisant à

des imidazolidinones de pKa supérieur ou égal à 5, on peut conduire une synthèse peptidique allant du C vers le N terminal, la fonction amine étant alors suffisamment nucléophile pour subir des réactions de couplage. Le point de départ est dans ce cas la fixation d'un α-aminoamide sur le support.

Au contraire, les polymères carbonylés conduisant à des imidazolidinones de pKa inférieur ou égal à 3 permettent de construire une synthèse peptidique allant du N vers le C terminal. En effet, dans ce cas, les fonctions amines des imidazolidinones, issues de dipeptides, sont suffisamment peu nucléophiles pour ne pas réagir vis-à-vis des réactifs de couplage, et ainsi ces imidazolidinones jouent parfaitement leur rôle de groupe protecteur de la fonction amine. On peut donc mener ce type de synthèse sans avoir à protéger la fonction amine par un groupe protecteur supplémentaire, ce qui simplifie considérablement la mise en oeuvre de cette synthese.

De plus, dans les deux cas, le décrochage du peptide final permet de régénérer automatiquement le polymère.

Selon une caractéristique particulière du procédé objet de la présente invention, le polymère carbonylé de départ est constitué par un polymère à motifs pipéridones, notamment une résine polyacryloylpipéridone, avantageusement réticulée par la N,N'-bis-acryloylpipérazine. Réticulée avec 20% de N,N'-bis-acryloylpipérazine, cette résine a une capacité d'environ 4 meq./g, des propriétés mécaniques satisfaisantes, ainsi que des taux de gonflement appréciables, par exemple une rétention de solvant $S_R = 4{,}03$ dans l'eau ; 2,98 dans le méthanol, 2,76 dans le dichlorométhane et 2,58 dans le diméthylformamide.

Selon une autre caractéristique particulière de la présente invention, le dipeptide, mis à réagir avec le polymère carbonylé de départ, répond à la formule générale suivante :

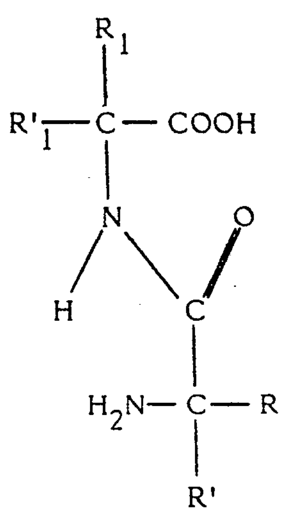

dans laquelle :

R et $R_1$ représentent indépendamment l'un de l'autre une chaîne latérale d'aminoacides naturels ou modifiés ;

R' représente un atome d'hydrogène ou bien R, et $R'_1$ représente un atome d'hydrogène ou bien $R_1$.

En particulier, chaque radical R et $R_1$ peut représenter une chaîne hydrocarbonée, linéaire ou ramifiée, contenant de 1 à 12 atomes de carbone et éventuellement un ou plusieurs hétéro-atomes, tels que le soufre, lesdits radicaux R et $R_1$ étant éventuellement substitués une ou plusieurs fois, de préférence en fin de chaîne, par des groupements tels que hydroxy, amino, carboxyle, phényle, hydroxyphényle, carboxamide, indolyle, iminazyle et guanidyle, ou bien R et $R_1$ forment avec l'atome d'azote en position α un groupe hétérocyclique saturé contenant au moins un hétéro-atome, tel que l'azote, ledit groupe hétérocyclique pouvant lui-même être substitué, par exemple par un groupe hydroxy.

Selon une autre caractéristique avantageuse de la présente invention, l'activation de la fonction acide terminale des cycles imidazolidinones fixés sur le polymère est obtenue par réaction avec le dicyclohexylcarbodiimide (DCC) en présence de N-hydroxybenzotriazole (HOBt).

En variante, cette activation peut également être obtenue par azidation qui correspond à une technique de couplage peptidique bien connue, décrite par CURTIUS T. (Ber. dtsch. Chem. Ges. - 1902, <u>35</u>, 3226).

Le dérivé peptidique à fonction amine libre et à fonction carboxyle protégée, utilisé au cours de l'étape de couplage (iii), est avantageusement constitué par un ester d'aminoacide ou de polypeptide.

Les exemples mentionnés ci-après illustrent la préparation de tripeptides par un procédé objet de la présente demande de brevet, sans pour autant en limiter la portée.

## EXEMPLE 1

### Préparation en phase solide de L-Val-Gly-L-PheOMe

#### a) Immobilisation de L-Val-Gly sur polyacryloylpipéridone :

Dans un réacteur thermostaté, on introduit 8,9 g de résine polyacryloylpipéridone 60-115 mesh réticulée avec 20% de N,N'-bis-acryloylpipérazine, $C_{pc=o} = 4$ meq$^t$./g. On ajoute ensuite 5,75 mmoles (1g) de L-valylglycine, solubilisées dans 22ml de méthanol. On porte avec agitation à 80°C pendant 3 heures. On sépare ensuite la résine par filtration, la lave à plusieurs reprises par le méthanol, ensuite successivement par $H_2O$, NaCl 1 M, $H_2O$, $CH_3OH$, $CH_2Cl_2$. On sèche ensuite la résine sous pression réduite ($10^{-2}$ mmHg). Sa capacité déterminée par dosage à l'hydroxylamine est d'environ 3 meq$^t$./g. Le taux de fixation est de 0,6 mmole par gramme de résine sèche.

#### b) Couplage de L-PheOMe

A 13,8 mmoles (2,9g) de L-PheOMe, HCl, on ajoute 14,3 mmoles (1,8ml) de N-éthylmorpholine, diluées dans 15 ml de $CH_2Cl_2$ anhydre. On sépare ensuite par filtration le chlorhydrate de la N-éthylmorpholine, puis additionne le filtrat sur la résine pipéridone Val-Gly préparée ci-dessus. On rajoute 13,8 mmoles (1,8g) de HOBt et 13,8 mmoles (2,8g) de DCC. On agite 24 heures à température ambiante, puis sépare la résine par filtration, et la lave successivement par $CH_2Cl_2$, $CH_3OH$, $CH_2Cl_2$, avant de la sécher sous pression réduite.

c) Hydrolyse

On réalise un montage en circuit fermé comportant deux colonnes, un réservoir tampon et une pompe.

Dans la première colonne thermostatée à 60°C, on place la résine pipéridone L-Val-Gly-L-PheOMe, dans la 2ème colonne non thermostatée, on introduit une résine sulfonique Duolite® (CF 204). Le tripeptide ester libéré par hydrolyse avec le mélange eau-méthanol 50/50 se fixe sur la résine sulfonique. Après 60 heures, on déconnecte la colonne de résine sulfonique, et par élution avec une solution d'ammoniaque 1N, on récupère le tripeptide brut. Après évaporation, puis lyophilisation, le résidu est agité pendant une nuit dans une solution HCl 5N dans le méthanol. Par addition d'éther anhydre on précipite ensuite 1,116g soit 3 mmoles de L-Val-Gly-L-PheOMe, HCl. F = 70°C (decomp.) [ $\alpha$ ]$_D$ = +45,1 (c= 1, DMF).

EXEMPLE 2

Préparation en phase solide de la L-alanyl-L-alanyl-L-alanine

A) Immobilisation de la L-alanyl-L-alanine

Selon la technique précédemment décrite pour immobiliser la L-Val-Gly, à partir de 6,24 mmoles (1g) de L-Ala-L-Ala dans 37ml de méthanol, on immobilise sur 10g de résine sèche 770mg de dipeptide, après 20h d'agitation à 60°C. Le taux de fixation est de 0,5mmole/g de résine sèche.

b) Couplage de l'ester benzylique de la L-alanine

Selon la méthode utilisée pour coupler le L-PheOMe sur la résine L-Val-Gly, on réalise le couplage à partir de 12,5 mmoles (2,696g) de L-Ala-OBz,HCl, 13,5 mmoles (1,9ml) de $NEt_3$, 12,5 mmoles (2,575g) de DDC et 12,5 mmoles (1,688g) de HOBt dans un mélange de $CH_2Cl_2$ et de DMF anhydres, en maintenant l'agitation 72 heures à température ambiante.

c) Hydrolyse

L'hydrolyse est réalisée suivant la technique utilisée pour libérer le tripeptide L-Val-Gly-L-PheOMe, dans l'eau distillée à 70°C. Après 4 jours, on déconnecte la colonne de résine sulfonique et, par élution avec une solution d'ammoniaque 1N, récupère le tripeptide brut ; on décèle des traces de dipeptide L-Ala-L-Ala en CCM, éluant $CH_3OH$-$NH_3$ 34% (95/5). Après évaporation de l'ammoniac sous pression réduite et lyophilisation, on élimine le dipeptide restant par chromatographie sur gel de silice, éluant $CH_3OH$-$NH_3$ 34% (95/5). Le produit est ensuite analysé en HPLC sur une colonne RP18 (Merck Lichrosorb), éluant $H_2O$ (1/1000 TFA), débit 1ml/mn, détection en UV à 210nm. Les temps de rétention sont pour l'épimère L-L-L de 2,76mn et pour l'épimère L-D-L de 6,77mn, pour une concentration en produit brut de 13,6mg dans 0,5ml d'eau distillée. Le taux d'épimérisation est de l'ordre de 1%.

## Revendications

1 - Procédé de préparation de polypeptides en phase solide, caractérisé en ce que l'on réalise successivement les étapes consistant à :

(i) Faire réagir un polymère carbonylé avec un dipeptide pour fixer ce dernier sur ledit polymère sous la forme de cycles imidazolidinones ;

(ii) Activer la fonction acide terminale portée par lesdits cycles imidazolidinones fixés sur le polymère ; et

(iii) Coupler les fonctions acides terminales activées portées par les cycles imidazolidinones du polymère issu de l'étape précédente, par réaction de ce dernier avec un dérivé peptidique à fonction amine libre et à fonction carboxyle protégée, puis

(iv) Hydrolyser le polymère issu de l'étape précédente pour séparer le polypeptide et régénérer simultanément le polymère carbonylé de départ.

2 - Procédé selon la revendication 1, caractérisé en ce que ledit polymère carbonylé de départ est un polymère à motifs pipéridones.

3 - Procédé selon la revendication 2, caractérisé en ce que ledit polymère à motifs pipéridones est une résine polyacryloylpipéridone, de préférence réticulée par la N,N'-bis-acryloylpipérazine.

4 - Procédé selon l'une des revendications 1 à 3, caractérisé en ce que le dipeptide mis à réagir avec le polymère carbonylé de départ répond à la formule générale suivante

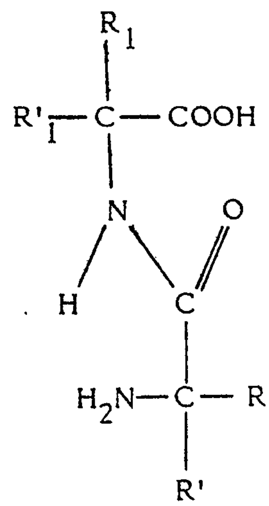

dans laquelle :

R et $R_1$ représentent indépendamment l'un de l'autre une chaîne latérale d'amino-acides naturels ou modifiés ;

R' représente un atome d'hydrogène ou bien R, et

R'$_1$ représente un atome d'hydrogène ou bien R$_1$.

5 - Procédé selon la revendication 4, caractérisé en ce que chaque radical R et R$_1$ peut représenter une chaîne hydrocarbonée, linéaire ou ramifiée, contenant de 1 à 12 atomes de carbone et éventuellement un ou plusieurs hétéro-atomes, tels que le soufre, lesdits radicaux R et R$_1$ étant éventuellement substitués une ou plusieurs fois, de préférence en fin de chaîne, par des groupements tels que hydroxy, amino, carboxyle, phényle, hydroxyphényle, carboxamide, indolyle, iminazyle et guanidyle, ou bien R et R$_1$ forment avec l'atome d'azote en position $\alpha$ un groupe hétérocyclique saturé contenant au moins un hétéro-atome, tel que l'azote, ledit groupe hétérocyclique pouvant lui-même être substitué, par exemple par un groupe hydroxy.

6 - Procédé selon l'une des revendications 1 à 5, caractérisé en ce que ledit peptide, mis à réagir avec le polymère carbonylé de départ, est choisi parmi

L-Val-Gly

et

L-Ala-L-Ala

7 - Procédé selon l'une des revendications 1 à 6, caractérisé en ce que l'activation de la fonction acide terminale des cycles imidazolidinones fixés sur le polymère est obtenue par réaction avec le dicyclohexylcarbodiimide (DCC) en présence de N-hydroxybenzotriazole (HOBt).

8 - Procédé selon l'une des revendications 1 à 6, caractérisé en ce que l'activation de la fonction acide terminale des cycles imidazolidinones fixés sur le polymère est obtenue par azidation.

9 - Procédé selon l'une des revendications 1 à 8, caractérisé en ce que le dérivé peptidique à fonction amine libre et à fonction carboxyle protégée utilisé lors de l'étape de couplage iii) est un ester d'aminoacide ou de polypeptide.

10 - Procédé selon la revendication 9, caractérisé en ce que ledit ester est choisi parmi l'ester méthylique de L-Phe et l'ester benzylique de L-Ala.

11 - Procédé selon l'une des revendications 1 à 10, caractérisé en ce que l'étape d'hydrolyse est obtenue par chauffage modéré à une température de l'ordre de 60°C en milieu hydro-alcoolique.

12 - Procédé selon la revendication 12, caractérisé en ce que le polypeptide est obtenu sous la forme d'un ester.

13 - Procédé selon la revendication 11, caractérisé en ce que l'ester de polypeptide obtenu est fixé sur une résine sulfonique.

14 - Procédé selon la revendication 13, caractérisé en ce que l'ester de polypeptide fixé sur la résine sulfonique est ensuite élué avec une solution d'ammoniaque pour conduire au tripeptide brut.